# EUROPEAN PATENT APPLICATION

(11) **EP 2 702 973 A1**
(43) Date of publication of application: **05.03.2014**
(21) Application number: 13182021.9
(22) Date of filing: 28.08.2013
(51) Int. Cl.: A61G 7/057, A61B 5/103

(54) **Interface pressure sensing mattress**

(30) Priority: 30.08.2012 US 201213598939
(71) Applicant: Hill-Rom Services, Inc., Batesville, IN 47006-9167 (US)
(72) Inventor: Jalbert, Ronald, North Charleston, SC South Carolina SC 29405 (US)
(74) Representative: Findlay, Alice Rosemary

(57) **Abstract**

A patient support surface or mattress has a coverlet defining an interior region and a core situated in the interior region. An interface pressure sensing sheet assembly is provided in the interior region. Above the core and beneath the coverlet, the sheet assembly has a lattice of longitudinally extending straps and laterally extending straps that are made of an interface pressure sensing material. The lattice has gaps through which the coverlet presses agaisnt the core. The laterally extending straps are provided over relatively high pressure zones of the core, such as a heel, sacral and/or occiput zone, and are not provided over other zones of the core.

## Description

The present disclosure relates to a patient support surface or mattress and particularly, to a device that is used with a mattress to sense interface pressure between a person and the mattress. More particularly, the present disclosure relates to an interface pressure sensing mattress that uses a layer of pressure sensing material to sense interface pressure.

Mats that are placed on top of a mattress to sense pressure between a person and the mattress are known. Electrical signals from these mats are sometimes use to create a graphical image or map of interface pressure on a display screen of a computer device. The map typically uses color to indicate ranges of interface pressure with red indicating regions of high pressure and other colors, such as shades of yellow or green indicating regions of lower pressure. The mat itself has a tendency to degrade the pressure performance of the mattress on which it is placed. As a result, these types of mats are used on mattresses only temporarily such as, for example, to compare pressure performances of various mattress products.

Some mattresses have pressure sensing devices situated internally, but these are oftentimes placed in the bottom portion of the mattress beneath one or more layers of primary support elements so as to minimize interference with the support characteristics of the overlying support elements. Some prior art does contemplate integrating interface pressure sensors at or near the top surface of a mattress, but it is understood that the interface pressure performance of the mattress is compromised in such systems. The known interface pressure sensing mats that are placed atop a mattress or integrated into the mattress near the upper surface generally occupy an uninterrupted square or rectangular area of space that extends substantially completely across the lateral dimension of the mattress. Some known interface pressure sensing systems are shown, for example, U.S. Pat. Nos. 7,973,666; 7,883,478; 7,825,814; and 7,557,718.

According to this disclosure, a patient support surface or mattress comprises one or more of the following features, alone or in any combination.

A mattress may have a coverlet defining an interior region. The mattress may also have a core situated in the interior region. The core may have at least one person support element. The core may have an upper surface, a pair of side surfaces, and a pair of end surfaces. The mattress further may have a sheet assembly having side portions that may fit closely against the pair of side surfaces of the core and end portions that may fit closely against the pair of end surfaces of the core within the interior region of the coverlet. The sheet assembly may have a plurality of longitudinally extending straps that may extend between the end portions and a plurality of laterally extending straps that may extend between the side portions. The longitudinally extending straps and laterally extending straps may form a lattice that may have a plurality of gaps through which the coverlet is able to press against the core. The longitudinally extending straps and laterally extending straps may be made of an interface pressure sensing material.

According to this disclosure, the interface pressure sensing material may include a highly stretchable, electrically conductive material. Furthermore, the interface pressure sensing material may include a breathable, piezoresistive material. In some embodiments, the sheet assembly may have a pair of side flaps that may extend atop the upper surface of the core adjacent the pair of side surfaces of the core and a pair of end flaps that may extend atop the upper surface of the core adjacent the pair of end surfaces of the core. End regions of the longitudinally extending straps may be fastened to the end flaps and end regions of the laterally extending straps may be fastened to the side flaps. Wiring may be coupled to at least some of the end regions of the longitudinally extending straps and at least some of the end regions of the laterally extending straps. The wiring may terminate at a connector that may be accessible outside the coverlet.

The core may be subdivided into a lower leg zone, an upper leg zone, a lower torso zone, and an upper torso zone. A first group of the laterally extending straps may be situated atop the lower leg zone and a second group of the laterally extending straps may be situated atop the lower torso zone. At least one or both of the upper leg zone and the upper torso zone may be devoid of any of the laterally extending straps thereabove. Thus, the laterally extending straps may be situated above some zones of the core but not others.

In some embodiments, the at least one person support element may include one or more foam elements. This disclosure contemplates that one or more of the foam elements may have at least one laterally extending slit and that the plurality of longitudinally extending straps may bridge across the one or more slits. Alternatively or additionally, one or more of the foam elements may have a plurality of holes extending vertically therethrough and at least some of the plurality of longitudinally extending straps may bridge across the holes.

Areas of overlap between the plurality of longitudinally extending straps and the plurality of laterally extending straps may be considered to form junctions. In some embodiments, the plurality of longitudinally extending straps and the plurality of laterally extending straps each may have substantially the same width such that the junctions may be square shaped. The plurality of longitudinally extending straps and the plurality of laterally extending straps may be electrically coupled at the junctions.
In another aspect, a mattress may have a coverlet defining an interior region. The mattress may also have a core situated in the interior region. The core may have at least one person support element. The core may have an upper surface, a pair of side surfaces, and a pair of end surfaces. The mattress further may have a sheet assembly having side portions that may fit closely against the pair of side surfaces of the core and end portions that may fit closely against the pair of end surfaces of the core within the interior region of the coverlet. The sheet assembly may have a plurality of longitudinally extending straps that may extend between the end portions and a plurality of laterally extending straps that may extend between the side portions. The longitudinally extending straps and laterally extending straps may form a lattice that may have a plurality of gaps through which the coverlet is able to press against the core. The longitudinally extending straps and laterally extending straps may be made of an interface pressure sensing material.

The core may have a first zone to support a first portion of a person and a second zone to support a second portion of the person. At least some of the plurality of laterally extending straps may be situated atop the first zone and the second zone may be devoid of any of the laterally extending straps thereabove. The first zone may include, for example, at least one or more of a heel support zone, a sacral support zone, and a head support zone. The second zone may include for example, at least one or more of a thigh support zone and a lower abdomen support zone. The longitudinally extending straps may be situated atop both the first and second zones.

The longitudinally extending straps and the laterally extending straps each may have end regions. Wiring may be coupled to at least some of the end regions of the longitudinally extending straps and at least some of the end regions of the laterally extending straps. The wiring may terminate at a connector that is accessible outside the coverlet. The connector may be coupled to another connector to communicate information pertaining to interface pressure to one or more of an electronic interface hub, a processing device such as a computer having a software platform, a local display device at a person's bedside, a remote display device such as one at a nurse's station, and a portable wireless communication device such as a pager, cellphone, personal digital assistant (PDA), and the like which may be carried by a caregiver.

The invention will now be further described by way of example with reference to the accompanying drawings, in which:

Fig. 1 is an exploded view of a mattress according to this disclosure showing a foam core and a sheet assembly with a lattice of interface pressure sensing straps situated above the foam core; and

Fig. 2 is a block diagram of a communication system over which interface pressure information is communicated to alert caregivers of interface pressure that exceed a threshold amount.

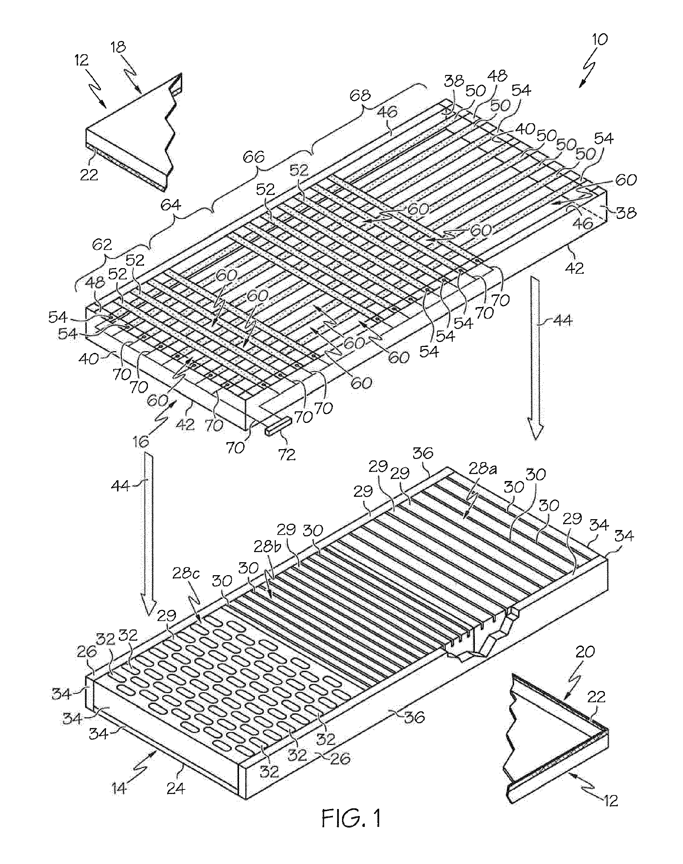

A patient support surface 10 has a coverlet 12, a core 14, and an interface pressure sensing sheet assembly 16 as shown in Fig. 1. Surface 10 is also referred to herein as mattress 10. Coverlet 12 includes an upper portion 18 that fastens to a lower portion 20 by suitable fasteners, such as zippers 22. While only some of upper and lower portions 18, 20 of coverlet 12 is shown in Fig. 1, it will be appreciated that coverlet 12 surrounds or encases core 14 and sheet assembly 16. Thus, core 14 and sheet assembly 16 are received in an interior region or space defined within coverlet 12. In some embodiments, mattress 10 includes a fire barrier layer (not shown), sometimes referred to as a "fire sock", which is well-known to those skilled in the art and which surrounds core 14 and sheet assembly 16 within the interior region of coverlet 12. In use, mattress 10 is supported on a bed frame (not shown).

Core 14 includes a number of person support elements. In the illustrative example, core 14 includes several foam elements including a base foam layer 24, a pair of side foam bolsters 26, and a set of foam blocks 28a-c. Foam blocks 28a-c rest atop base foam layer 24 and are situated between foam bolsters 26. In some embodiments, adhesive is used to fasten elements 24, 26, 28a-c of core 14 together. Upper surfaces 29 of foam blocks 28a-c and bolsters 26 are generally coplanar as shown in Fig. 1. Foam blocks 28a, 28b each include a plurality of slits 30 that extend laterally across the upper surfaces 29 thereof. Foam block 28c has a number of holes 32 that extend vertically therethrough. In the illustrative example, holes 32 are generally oval in shape with their long dimensions being oriented parallel with the lateral dimension of mattress 10. Core 14 has end surfaces 34 and side surface 36. End surfaces 34 and side surfaces 36 are oriented substantially vertically when core 14 is laid out flat as shown in Fig. 1.

Sheet assembly 16 is akin to a fitted sheet in that it includes side portions 38 that fit closely against the pair of side surfaces 36 of core 14 and end portions 40 that fit closely against the pair of end surfaces 34 of the core 14 within the interior region of the coverlet 12. A resiliently stretchable cord or band (not shown) is provided at the bottom edge 42 of portions 38, 40 of sheet assembly 16 in some embodiments. The stretchable cord or band at the bottom edge 42, which may be made from elastic material, for example, helps to retain sheet assembly 16 on core 14. Alternatively or additionally, corner straps that extend diagonally between the bottom edge 42 of portions 38 and the bottom edge 42 of portions 40 in the corner regions of sheet assembly 16 are provided to help retain sheet assembly 16 on core 14. Thus, sheet assembly 16 has an open bottom and, during assembly, sheet assembly 16 is moved downwardly over core 14 as indicated by arrows 44 in Fig. 1.

In the illustrative example of Fig. 1, sheet assembly 16 has a pair of side flaps 46 that extend atop the upper surface 29 of the core 14 adjacent the pair of side surfaces 36 of the core 14 and a pair of end flaps 48 that extend atop the upper surface 29 of the core 14 adjacent the pair of end surfaces 34 of the core 14. Flaps 46, 48 are basically upper extensions of side and end portions 38, 40 of sheet assembly 16 that are folded over at roughly a right angle. Sheet assembly 16 has a plurality of longitudinally extending straps 50 that extend between the end portions 40 of sheet assembly 16 and a plurality of laterally extending straps 52 that extend between the side portions 38. Straps 50, 52 each have end regions 54 that are fastened to respective flaps 46, 48 with suitable fasteners such as, for example, adhesive, stitching, radio frequency (RF) or sonic welds, snaps, rivets, and the like.

According to this disclosure, straps are made of interface pressure sensing material that is highly stretchable and, in some cases, breathable. The term "highly stretchable" is intended to mean that the material of straps 50, 52 is able to stretch to a greater extent than the underlying foam elements 24, 26, 28a-c are able to stretch and more than the surrounding coverlet 12 is able to stretch. Thus, straps 50, 52 easily deform under the weight of a person lying on mattress 10 so as to minimize interference with the pressure support performance of the underlying core 14. Examples of such highly stretchable material include LYCRA and SPANDEX materials. Thus, highly stretchable materials contemplated by this disclosure will usually stretch 20% or more in length and 8 to 10% or more in width. In some embodiments, the contemplated highly stretchable material may stretch about 60-120% in length and about 40% in width and still be within its elastic limits. However, materials with less stretchability may be satisfactory for straps 50, 52.

Straps 50, 52 comprise a fabric with piezoresistive material bonded to at least one other layer of the straps 50, 52 in the illustrative example. Thus, straps 50, 52 are electrically conductive and the impedance, such as the resistivity, of straps 50, 52 changes based on the force applied to the straps 50, 52 and the amount that the straps 50, 52 are stretched. However, while electrically conductive, straps 50, 52 are non-metallic in nature. The type of material that comprises straps 50, 52 is available from companies such as Eeonyx Corporation of Santa Clara, California under the brand name EEONTEX. Suitable materials may be available from other suppliers as well. See also U.S. Patent Nos. 6,543,299; 7,468,332; and 8,161,826; each of which is hereby incorporated by reference herein, for additional details regarding the type of material from which straps 50, 52 may be made.

As is evident in the example of Fig. 1, straps 50, 52 of sheet assembly 16 are arranged to form a lattice having a plurality of gaps 60 through which the coverlet 12 is able to press against upper surface 29 of core 14. In those embodiments of mattress 10 having a fire sock (not shown) or other layer(s) of material interposed between coverlet 12 and straps 50, 52 of sheet assembly 16, the coverlet 12 is still considered to be pressing against core 14 through gaps 60 according to this disclosure even though it is doing so through the fire sock or other layer(s).

The lattice arrangement of straps 50, 52 with the large number of gaps 60 results in a substantial portion of the surface area of upper surface 29 of core being exposed in the gaps 60. In some embodiments, such as the illustrative embodiment, the spacing between each of straps 50 and between each of straps 52 is equal to or greater than the width of the straps. The width of the straps may be on the order of about 0.5 to about 2 inches in some embodiments. However, greater or lesser widths of straps 50, 52 are contemplated by this disclosure. In any event, a large portion of upper surface 29 is completely unobstructed by straps 50, 52 thereby further minimizing any interference with the pressure support performance of core 14 by straps 50, 52. This is an improvement over prior art pressure sensing mats that cover the entire upper surface of a mattress and an improvement over smaller mats that, while covering less of a mattress, still usually cover an entire zone of the mattress.

Another feature of the illustrative embodiment is that the laterally extending straps 52 are provided in some zones of mattress 10 and not others. For example, core 14 as well as mattress 10 itself, may be considered to have a lower leg or heel zone 62, an upper leg or thigh zone 64, a lower torso or sacral zone 66, and an upper torso or head/shoulder zone 68 as shown in Fig. 1. In the illustrative embodiment, straps 52 are situated atop zones 62, 66 of core 14 but not above zones 64, 68. In other embodiments, additional straps 52 are provided in a sub-portion of zone 68 on which a patient head rests. This sub-portion may be referred to as the occiput zone. It is within the scope of this disclosure, to have straps 52 included in all zones 62, 64, 66, 68 of mattress 10, if desired. However, providing straps 52 only above the zones of core 14 where a person's interface pressure with the mattress tends to be the highest (e.g., heels, sacrum, and occiput) results in interference with the pressure support performance of core 14 in the other zones being further minimized.

In the illustrative embodiment, the longitudinally extending straps 50 extend over all of zones 62, 64, 66, 68 of core 14. Furthermore, straps 50 bridge across the slits 30 of foam blocks 28a, 28b and bridge across some of the holes 32 of foam block 28c. Some of laterally extending straps 52 may lay above certain slits 30 and holes 32 as well.

According to this disclosure, areas of overlap between the plurality of longitudinally extending straps 50 and the plurality of laterally extending straps 52 form junctions. In some embodiments, straps 50, 52 may be fastened together at these junctions such as by stitching, adhesive, RF or sonic welding, and the like. In the illustrative example, however, straps 50, 52 are not physically coupled at the junctions. Because straps 50, 52 are electrically conductive as mentioned above, the straps 50, 52 are electrically coupled at the junctions regardless of whether or not they are physically coupled at the junctions. In the illustrative example, all of straps 52 overlie straps 50. However, other arrangements, such as having some or all of straps 52 underlying straps 50 or forming a basket weave type of pattern with straps 50, 52 are within the scope of this disclosure.

Mattress 10 includes wiring 70 which has a number of conductors, each conductor of wiring 70 being coupled to one of the end regions 54 of each of straps 50, 52 as shown in Fig. 1. Thus, electrical paths pass through each of the junctions between straps 50, 52 and application of electrical potential can be multiplexed to the array or matrix of electrical paths via the conductors of wiring 70 so that current flows through a selected one of straps 50 and a selected one of straps 52. The wiring terminates at a connector 72 that is accessible outside coverlet 12.

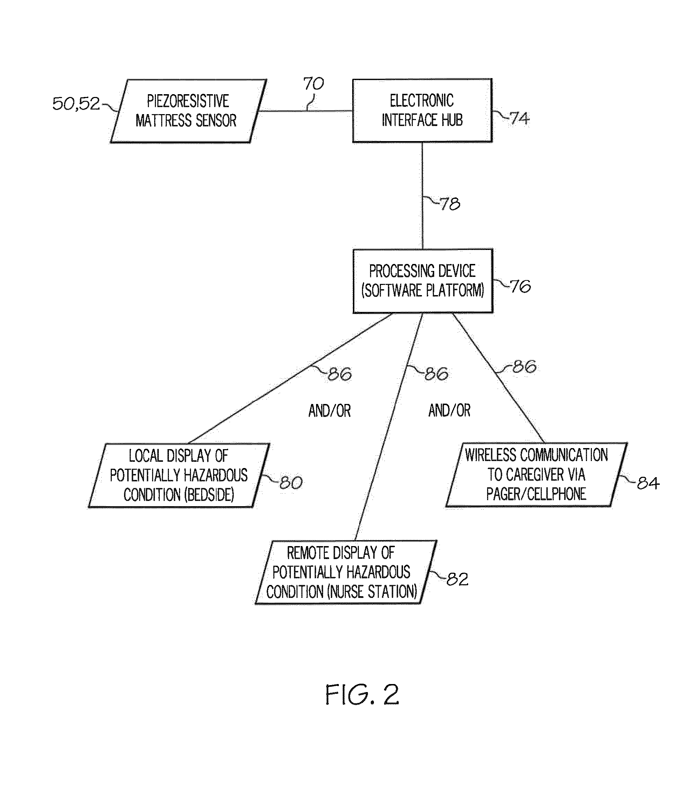

Referring now to Fig. 2, wiring 70 from straps 50, 52 (collectively labeled a block in Fig. 2 as "Piezoresistive Mattress Sensor") connects to an electronic interface hub 74. Thus, hub 74 includes a connector or jack (not shown) that is configured to mate with connector 72 at the end of wiring 70. Hub 74 is, in turn, coupled to a processing device 76 via a data and/or communication link 78. Device 76 includes computer hardware and software to control the application of voltage and current to the array or matrix formed by straps 50, 52 and to determine the interface pressure based on the voltage drop or current flow through the selected electrical path of straps 50, 52. Based on that information, an interface pressure table, graph, and/or map is generated by device 76 for storage, analysis, communication and/or display. For example, in Fig. 2, block 80 represents local display on a display device of an alarm message in response to the analysis by the software of device 76 indicating that a hazardous condition has been detected. The local display device also shows the pressure graph or map in some embodiments. Remote display of this same type of alarm message or graph or map is also contemplated as indicated by block 82 in Fig. 2. In some embodiments, device 76 may initiate wireless communication to a portable wireless communication device, such as a pager or cellphone carried by one or more caregivers as indicated by block 84 in Fig. 2.

Communication links are shown diagrammatically as lines 86 between device 76 and each of blocks 80, 82, 84 in Fig. 2. Lines 86 are intended to represent the various components in the electrical architecture of a network of a healthcare facility, such as a hospital or nursing home. The electrical architecture includes components such as servers, routers, wireless access points, electrical cables, wires, electrical jacks, and so forth, just to name a few.

In some embodiments, the software of device 76 is programmed to monitor interface pressure over a predetermined or specified time limit. In addition to generating an alarm message in response to interface pressure exceeding a programmed threshold, it is also contemplated by this disclosure that an alarm message is generated if patient inactivity occurs for a threshold amount of time. Patient inactivity is determined based on the information from sheet assembly 16. That is, if the interface pressures are stagnant or don't change by some threshold amount over the predetermined period of time, then the patient is considered to be inactive and should potentially be repositioned by one or more caregivers. In some embodiments, audible alarms may also be generated in addition to displaying visual alarms on the local and/or remote display devices. It is contemplated by this disclosure that caregivers are able to view pressure graphs or maps on the local and/or remote displays even when alarm conditions are not occurring. Thus, during the absence of alarm conditions, caregivers have the ability to monitor substantially real time pressure maps of patient position to decide whether early intervention may prevent hazardous conditions from developing.

Although certain illustrative embodiments have been described in detail above, many variations and modifications are possible.

## Claims

1. A mattress comprising
a coverlet defining an interior region,
a core situated in the interior region, the core having at least one person support element, the core having an upper surface, a pair of side surfaces, and a pair of end surfaces, and
a sheet assembly having side portions that fit closely against the pair of side surfaces of the core and end portions that fit closely against the pair of end surfaces of the core within the interior region of the coverlet, the sheet assembly having a plurality of longitudinally extending straps that extend between the end portions and a plurality of laterally extending straps that extend between the side portions, the longitudinally extending straps and laterally extending straps forming a lattice in at least one region of the sheet assembly, the lattice having a plurality of gaps through which the coverlet is able to press against the core, the longitudinally extending straps and laterally extending straps being made of an interface pressure sensing material.

2. The mattress of claim 1, wherein the core has a lower leg zone, an upper leg zone, a lower torso zone, and an upper torso zone, wherein a first group of the laterally extending straps are situated atop the lower leg zone and a second group of the laterally extending straps are situated atop the lower torso zone, and wherein at least one of the upper leg zone and the upper torso zone are devoid of any of the laterally extending straps thereabove.

3. The mattress of claim 2, wherein the upper leg zone and the upper torso zone are both devoid of any of the laterally extending straps.

4. The mattress of claim 1
wherein the core has a first zone to support a first portion of a person and a second zone to support a second portion of the person, at least some of the plurality of laterally extending straps being situated atop the first zone and the second zone being devoid of any of the laterally extending straps thereabove.

5. The mattress of claim 4, wherein the first zone comprises at least one of a heel support zone, a sacral support zone, and a head support zone.

6. The mattress of claim 5, wherein the second zone comprises at least one of a thigh support zone and a lower abdomen support zone.

7. The mattress in any one of claims 4 to 6, wherein the longitudinally extending straps are situated atop both the first and second zones.

8. The mattress of any preceding claim, wherein the longitudinally extending straps and the laterally extending straps each have end regions and further comprising wiring coupled to at least some of the end regions of the longitudinally extending straps and at least some of the end regions of the laterally extending straps.

9. The mattress of claim 8, wherein the wiring terminates at a connector that is accessible outside the coverlet.

10. The mattress of any preceding claim, wherein the sheet assembly further comprises a pair of side flaps that extend atop the upper surface of the core adjacent the pair of side surfaces of the core and a pair of end flaps that extend atop the upper surface of the core adjacent the pair of end surfaces of the core, wherein end regions of the longitudinally extending straps are fastened to the end flaps, and wherein end regions of the laterally extending straps are fastened to the side flaps.

11. The mattress of any preceding claim, wherein the interface pressure sensing material comprises a highly stretchable, electrically conductive material.

12. The mattress of claim 11, wherein the interface pressure sensing material comprises a breathable, piezoresistive material.

13. The mattress of any preceding claim, wherein the at least one person support element comprises at least one foam element.

14. The mattress of claim 13, wherein at least one foam element has a laterally extending slit and the plurality of longitudinally extending straps bridge across the slit.

15. The mattress of either claim 13 or claim 14, wherein at least one foam element has a plurality of holes extending vertically therethrough and wherein at least some of the plurality of longitudinally extending straps bridge across at least some of the plurality of holes.
